# EUROPEAN PATENT SPECIFICATION

(11) **EP 3 235 899 B1**
(45) Date of publication and mention of the grant of the patent: **26.04.2023**
(21) Application number: 16000873.6
(22) Date of filing: 18.04.2016
(51) Int. Cl.: C12M 1/12, B01L 3/00

(54) **METHOD OF PRODUCING 3D CELL STRUCTURES IN HANGING DROPLETS AND HANGING DROPLET DEVICE COMPRISING 3D CELL STRUCTURES**
VERFAHREN ZUR HERSTELLUNG VON 3D-ZELLSTRUKTUREN IN HÄNGENDEN TROPFEN UND HÄNGENDE TROPFVORRICHTUNG MIT DEN 3D-ZELLSTRUKTUREN
PROCÉDÉ DE PRODUCTION DE STRUCTURES CELLULAIRES 3D DANS DES GOUTTELETTES DE SUSPENSION ET DISPOSITIF DE GOUTTELETTES DE SUSPENSION 3D COMPRENANT DE TELLES STRUCTURES

(43) Date of publication of application: 25.10.2017
(73) Proprietor: Karlsruher Institut für Technologie, 76131 Karlsruhe (DE)
(72) Inventor: Demir, Konstantin, 76287 Rheinstetten (DE); Popova, Anna, 76149 Karlsruhe (DE); Levkin, Pavel, 76344 Eggenstein (DE)
(74) Representative: Müller-Boré & Partner Patentanwälte PartG mbB

(56) References cited:
- EP-A1- 2 684 601
- US-A1- 2013 084 634

## Description

The present invention relates to a method of producing 3D cell cultures. Further, the present invention relates to a hanging droplet device comprising hanging separated homogeneous aqueous fluid microdroplets which comprise 3D cell structures, and a use of the hanging droplet device for high-throughput screening and for cultivating 3D cell structures.

High-throughput (HT) screening of live cells represents a powerful tool for the development of novel therapeutics in the pharmaceutical industry. Currently, *in vitro* studies are mostly carried out using two-dimensional (2D) cell culture systems. However, three-dimensional (3D) *in vitro* models or cell structures, for example spheroids, can resemble tissue from eukaryotic organisms (like organ or tumor tissue) in a way that drug testing and screenings based on said 3D cell structures yield more therapeutically predictive relevant results. In addition to the advantage of increasing the predictability, 3D cell culture systems enable the reduction of animal experiments.

So far the production of 3D cell structures using the method of hanging droplets depends upon special platforms which require expensive and specialized equipment such that the production of 3D cell structures is quite costly. Such platforms exist mostly in 96- and sometimes in 384-well formats and do not offer high throughput applications. In addition, high droplet volumes are used, which reflects in high reagent consumption. Finally, the seeding of cells and further manipulations such as medium exchange is performed manually and is therefore time consuming.

A promising approach for conducting HT screening of live cells has been developed by the present inventors in EP 2 684 601 A1, which discloses a method of forming an array of separated homogenous fluid microdroplets or hydrogel micropads of a desired shape and size in a desired spatial pattern, comprising the steps of providing a patterned substrate with a solid support coated with a porous polymer layer or film comprising hydrophilic areas having the desired shape and size, surrounded by hydrophobic areas separating the hydrophilic areas into the desired spatial pattern, and applying the fluid to a multitude of the hydrophilic areas at the same time. However, EP 2 684 601 A1 does not relate to 3D cell structures such as spheroids, organoids or embryonic bodies at all. Regarding 3D models, it is only referred to cultivating cells in hydrogels. Based on its framework structure, hydrogel provides a three-dimensional environment which enables the cells to proliferate in a more natural way compared to the proliferation in 2D cell cultures. However, 3D cell structures such as spheroids, organoids or embryonic bodies are very different from the cells cultivated in a hydrogel. In particular, 3D cell structures such as spheroids, organoids or embryonic bodies result from the agglomeration of adherent cells which assemble to three-dimensional cell structures based on cell-cell contacts. On the contrary, hydrogels do not provide such 3D cell structures, but only a 3D environment for the proliferation of cells. Additionally, in the invention described in EP 2 684 601 A1, a hanging droplet technique is mentioned as a potential beneficial method to culture non-adherent cells that are not forming 3D cell structures such as spheroids, organoids or embryonic bodies. In contrast, in the present invention a method employing hanging droplets is proposed for the formation of 3D cell structures such as spheroids, organoids or embryonic bodies from the agglomeration of adherent cells.

In particular, there is a need for a method which enables a cost-efficient production of 3D cell structures such as spheroids, organoids or embryonic bodies, which can be used in HT screenings applications, as well as for a device which comprises 3D cell structures such as spheroids, organoids or embryonic bodies and can be used in HT screening applications.

Therefore, the technical problem underlying the present invention is to provide a method of producing 3D cell structures such as spheroids, organoids or embryonic bodies as well as a device comprising 3D cell structures such as spheroids, organoids or embryonic bodies and the use of said device for high-throughput screening of the 3D cell structures such as spheroids, organoids or embryonic bodies and for cultivating them.

The solution to the above technical problem is achieved by the embodiments characterized in the claims.

In particular, the present invention relates to a method of producing 3D cell structures, comprising the steps of:
(1) forming an array of separated homogenous aqueous fluid microdroplets of a desired shape and size in a desired spatial pattern, wherein the separated homogenous aqueous fluid microdroplets comprise cells, wherein the forming of the array comprises:
   (a) providing a patterned substrate with a solid support coated with a layer or film comprising
      (i) hydrophilic areas having the desired shape and size; surrounded by
      (ii) hydrophobic areas separating the hydrophilic areas into the desired spatial pattern;
         and
   (b) applying the aqueous fluid comprising the cells to a multitude of the hydrophilic areas at the same time;
(2) turning the patterned substrate with the solid support upside down such that the separated homogeneous aqueous fluid microdroplets form hanging separated homogeneous aqueous fluid microdroplets;
(3) agglomerating the cells comprised in the hanging separated homogeneous aqueous fluid microdroplets to form 3D cell structures,
   and
   further comprising a step (2') between step (2) and (3):
   (2') providing the turned patterned substrate with the solid support comprising the hanging separated homogeneous aqueous fluid microdroplets on a holder, wherein the holder comprises a plate having an opening which is adjusted to hold the turned patterned substrate with the solid support;
   wherein a cell-culture dish comprising a liquid is provided below the holder,
   wherein the volume of one hanging separated homogenous aqueous fluid microdroplet is in the range from 5 nL to 100 nL, and
   wherein the holder is a table comprising a tabletop with an opening which is adjusted to hold the turned patterned substrate with the solid support and comprises legs which extend from the surface of the plate facing downwards to the ground.

According to the present invention, the 3D cell structures are not particularly limited and can be selected from spheroids, organoids, and embryonic bodies, with the proviso that the embryonic bodies are not human embryonic bodies and no human embryo is destroyed in the method. In a preferred embodiment of the present invention, the 3D cell structures are spheroids.

The term "aqueous fluid" as used herein is not particularly limited and relates to any aqueous fluids that might be of interest for the formation of a respective microdroplet array. In particular, the aqueous fluid can be selected from the group consisting of water, aqueous solutions and aqueous media.

According to the present invention, the aqueous fluid applied to the hydrophilic areas comprises cells. The term "cell" as used in this context is not particularly limited and encompasses any kind of prokaryotic and eukaryotic cells that might be of interest. Preferably, the cells are selected from at least one member of a group consisting of human, animal or plant primary cells, immortalized adherent and non-adherent cell lines, human and animal cancer cells and, human and animal stem cells. In order to enable the agglomeration of 3D cell structures, the presence of adherent or semi-adherent cells is required. However, co-cultures with non-adherent cells such as immune cells can be used for a better representation of an *in vivo* environment. Such 3D cell structures are not restricted to spheroids and can apply to organoids in case of co-culturing of multiple cell types, and to embryonic bodies in case of culturing of stem cells. For producing organoids, non-cancer cells, such as cerebral cells, hepatic cells, pancreatic cells, gastric cells, epithelial cells, lung cells, and kidney cells, can be used. According to a preferred embodiment of the present invention, cancer cells may be used which agglomerate to miniaturized tumors. The cancer cells are not particularly limited to one cell type and may be selected from any existing cancer type which forms solid tumors.

The term "microdroplet" as used herein refers to the droplets formed in the method of the present invention. Patterns confining the microdroplets can have any desired shape, e.g. a circular or hexagonal shape, and can have also sharp-edged geometries such as triangular shapes. The patterns can have dimensions of 50 µm to 1 cm, and one microdroplet has a volume from 5 nL to 100 nL, more preferably 20 nL to 100 nL. The number of microdroplets formed in the method of the present invention is not particularly limited and depends on the size of the patterned substrate and the number of hydrophilic areas formed thereon. As an example, arrays, in particular high-density arrays, of thousands of microdroplets can be formed. The microdroplets are preferably homogenous, i.e. they all have the same size and shape, and they are separated, i.e. they are separated from each other by the hydrophobic areas separating the hydrophilic areas on which the microdroplets form. In this manner, the individual microdroplets are completely isolated. An array of microdroplets as formed in the method of the present invention is sometimes referred to as "DropletArray" hereinafter.

The term "spatial pattern" as used herein is not particularly limited and can encompass any desired pattern of microdroplets. Preferably, the pattern is chosen in a way that the density of microdroplets per area is maximized.

In the following, the terms "area" and "spot" will be used synonymously. As an example, the hydrophilic areas of the patterned substrates used in the present invention can be referred to as hydrophilic spots or just as spots.

According to the present invention, the term "3D cell structures" refers to 3D aggregates or clusters of cells and/or cell colonies, which might include spheroids, organoids and embryonic bodies. 3D cell structures may be formed from various cell types such as human, animal or plant primary cells, immortalized adherent and non-adherent cell lines, human and animal cancer cells and, human and animal stem cells.

In the present invention, the 3D cell structures are formed from the cells comprised in the aqueous fluid applied to the hydrophilic areas in step (b). The shape of a 3D cell structure may be sphere-like or irregular. The 3D cell structures may comprise heterogeneous populations of cells represented by various cell types, as well as cells in different states, such as for example proliferating cells, quiescent cells, and necrotic cells.

The size of the 3D cell structures is not particularly limited and depends on the size of the hydrophilic spots, which can be controlled by the design of a photomask that is used for producing the patterned substrate, and on the cell concentration used in the aqueous fluid. According to a preferred embodiment of the present invention, the size of one 3D cell structure is from 20 to 700 µm, preferably, from 100 to 300 µm and, more preferably, from 150 to 200 µm.

The 3D cell structures are preferably located in the lower end of the hanging separated homogenous aqueous fluid microdroplets. In that position the 3D cell structures do not have contact to the surface of the substrate such that adhesion between surface and 3D cell structures is avoided.

The number of 3D cell structures per hanging separated homogeneous aqueous fluid microdroplet is not particularly limited. According to a preferred embodiment, each of the hanging separated homogenous aqueous fluid microdroplets comprises one 3D cell structure. The presence of only one 3D cell structure per hanging separated homogeneous aqueous fluid microdroplet is advantageous in biological assays and applications.

Step (b) of the method of the present invention, *i.e.* the step of applying the aqueous fluid comprising the cells to a multitude of the hydrophilic areas at the same time is a step wherein the aqueous fluid comprising the cells is applied at the same time to at least more than one, preferably more than 5, 10, 20, 50, 100, 500, or 1000 spots, more preferably to all spots. Alternatively, the aqueous fluid is applied at the same time to at least 5, 10, 15, 20, 30, 50, or 75% of all spots, more preferably to 100% of the spots.

In particular, the aqueous fluid is preferably not applied on a spot-by-spot basis.

According to a preferred embodiment of the present invention, the aqueous fluid can be applied manually or with the help of an automated liquid-handling device. In a more preferred embodiment, the aqueous fluid is applied manually with the help of a pipette or with the help of an automated machine by spreading or rolling an amount of the aqueous fluid comprising the cells along the surface of the patterned substrate. The amount of fluid used in this particular embodiment is preferably an amount that is just sufficient to form microdroplets on all hydrophilic spots of the patterned substrate. The patterned substrate can also be immersed, manually or with the help of an automated machinery, into a solution or suspension of cells to form a Droplet-Microarray. Using automated dispensers of any kind to spot substances or cells on individual hydrophilic spots is not excluded and can be used if it is in favor of an application.

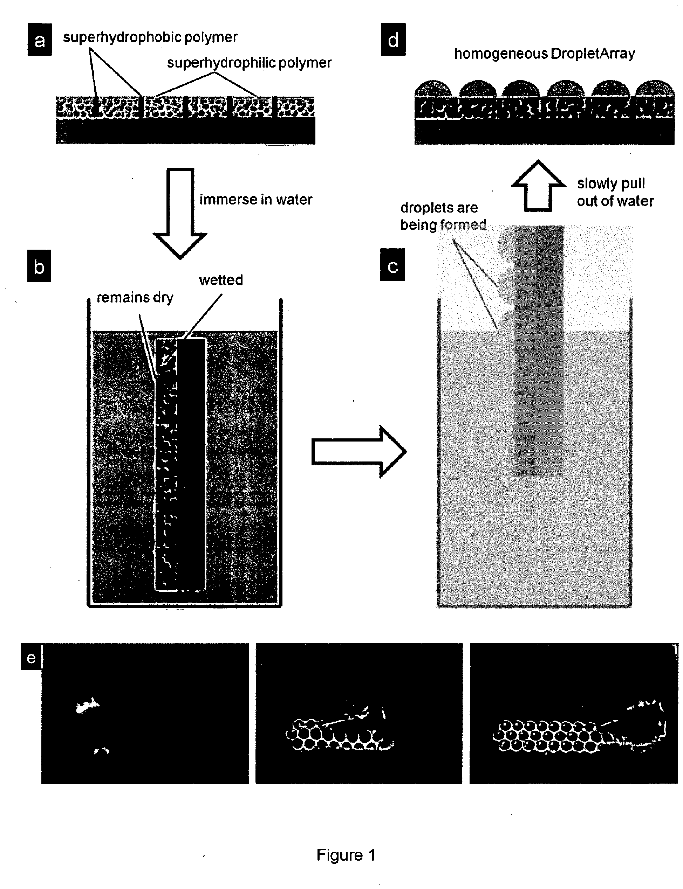

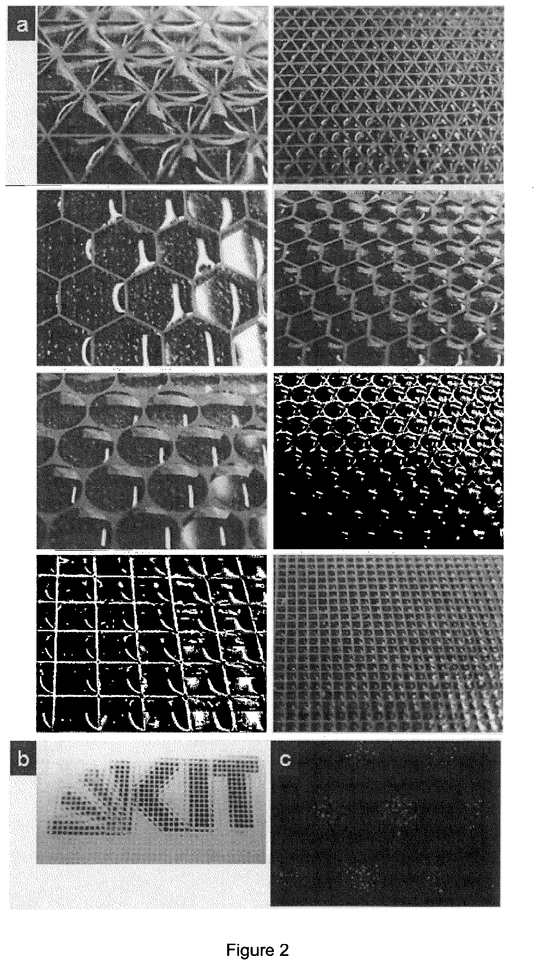

Embodiments of the application of the aqueous fluid is shown in Fig. 1. Further, images of arrays of droplets formed with the method of the present invention are shown in Fig. 2.

The patterned substrate used in the method of the present invention is preferably a microarray, more preferably a cell microarray. Further, said patterned substrate preferably has a flat surface, and does not comprise any physical barriers, such as wells, recesses, indentations, depressions, ridges or the like, separating the individual hydrophilic areas from each other.

The layer or film coated on the patterned substrate is not particularly restricted as long as said layer or film can provide hydrophilic and hydrophobic regions. According to a preferred embodiment, the layer or film used in the present invention is a porous polymer layer or film coated on the patterned substrate. Preferably, the porous polymer layer or film is biocompatible, *i.e.* it does not impair or negatively affect the growth, proliferation or well-being of the cells or 3D cell structures that are trapped in the microdroplets formed, and does not impair the structural or functional integrity of any biologically active compounds, such as nucleic acids, peptides, proteins and enzymes, that may be present in the microdroplets.

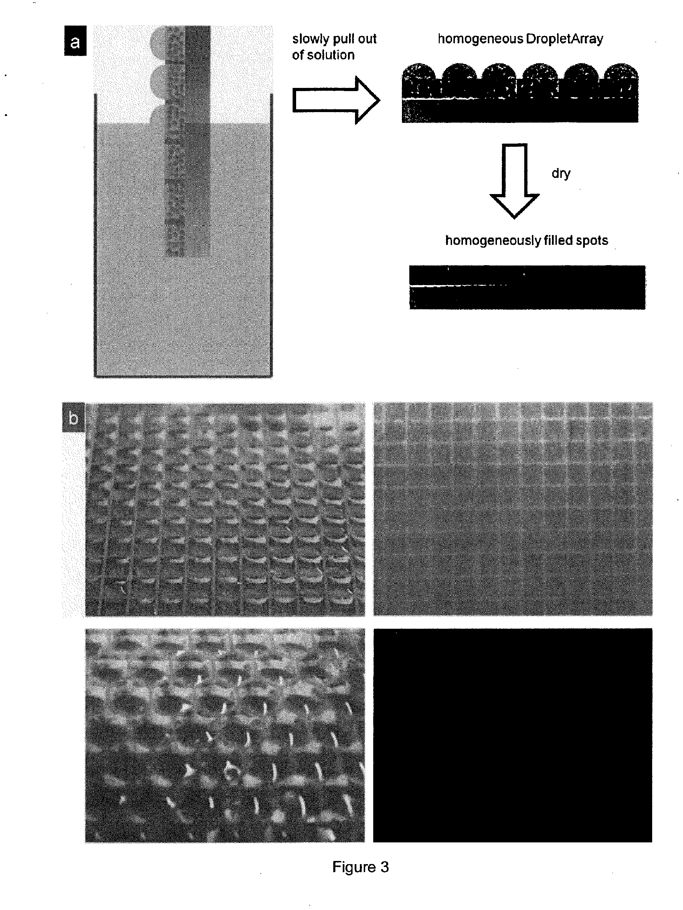

In preferred embodiments, at least one compound is added into individual aqueous fluid microdroplets formed on the patterned surface. Preferably, the at least one compound is selected from the group consisting of chemical compounds, pharmaceutically active agents, biologically active agents, toxins, nucleic acids, peptides, proteins, enzymes, cross-linkers, buffer substances, water-soluble polymers and mixtures thereof. Methods for addition of any of said compounds to the hydrophilic areas are not particularly limited and include for example the formation of microdroplets of an aqueous fluid and addition of the compounds to the droplets using a sandwiching approach. Alternatively, an aqueous fluid containing at least one of the above compounds can be applied to the patterned substrate to form microdroplets. In a subsequent step, the microdroplets can be dried. After further adding the aqueous fluid comprising cells, the compounds contained in the spots of the pattern can be absorbed into the newly formed droplets comprising the cells. A schematic overview of the application of a fluid containing at least one of the compounds and subsequent drying is exemplified with the deposition of the dye methyl green in Fig. 3.

Step (2) of the method of the present invention, *i.e.* turning the patterned substrate with the solid support upside down such that the separated homogeneous aqueous fluid microdroplets form hanging separated homogeneous aqueous fluid microdroplets is a step wherein the patterned substrate is preferably turned manually. The term "upside down" as used herein refers to a state wherein the patterned substrate is turned by about 180 degrees such that the patterned substrate faces down after the turning step. By turning the patterned substrate, the separated homogeneous aqueous fluid microdroplets form hanging separated homogeneous aqueous fluid microdroplets. Upon this turning step, hanging separated homogeneous aqueous fluid microdroplets are created which do not provide a surface to the cells comprised in the aqueous fluid to which they can adhere to. Therefore, an additional surface coating which prevents the cells from adhering to the surface is not necessary. In particular, by turning the patterned substrate, the cells comprised in the microdroplets sink preferably to the lower end of the hanging separated homogeneous aqueous fluid microdroplets.

Further, step (3) of the method of the present invention, *i.e.* agglomerating the cells comprised in the hanging separated homogeneous aqueous fluid microdroplets to 3D cell structures is a step wherein the 3D cell structures are formed by self-assembly of the cells.

The method according to the present invention comprises a further step (2') between step (2) and (3):
(2') providing the turned patterned substrate with the solid support comprising the hanging separated homogeneous aqueous fluid microdroplets on a holder, wherein the holder comprises a plate having an opening which is adjusted to hold the turned patterned substrate with the solid support.

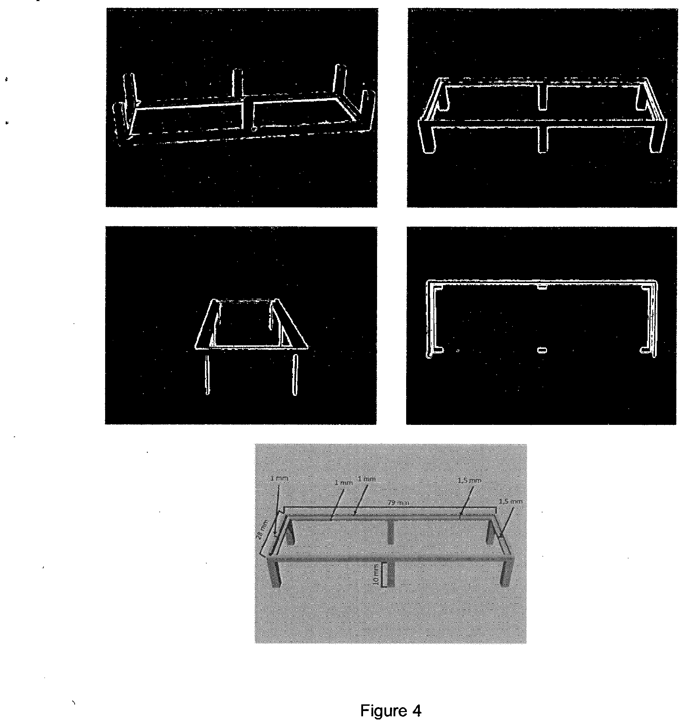

A preferred embodiment of the holder is shown in Figure 4. The holder is a table comprising a tabletop with an opening which is adjusted to hold the turned patterned substrate with the solid support and comprises legs which extend from the surface of the plate facing downwards to the ground. More preferred, the holder comprises six legs, one on each corner and additionally one in the middle of each long side. The height of the holder is not particularly limited and is determined by the length of the legs. In a preferred embodiment, the height of the holder is at least 6 mm, preferably at least 8 mm, and more preferably 10 mm. According to a preferred embodiment of the present invention, the holder supports the turned patterned substrate along the perimeter of the substrate with its frame. Preferably, the supporting frame of the holder is constituted by the edges of the plate which surround the opening of said plate. By providing the turned patterned substrate onto the holder, contact between the hanging separated homogeneous aqueous fluid microdroplets and the ground can be avoided. The size of the holder is not particularly limited and depends on the size of the patterned substrate. Preferably, the holder has a size which allows placing it into a standard 10 cm cell culture dish. According to a preferred embodiment, the plate of the holder has about the same size as the patterned substrate, which can be a standard microscope glass slide.

Providing the turned patterned substrate on the holder and removing it from the holder is an easy handling step and can for example be performed manually. Thereby it is possible to keep the turned patterned substrate mobile which is advantageous in view of analyses with a microscope or performing of tests on the 3D cell structures comprised in the hanging separated homogeneous aqueous fluid microdroplets.

The material of the holder is not particularly limited as long as it is sufficiently stable to support the turned patterned substrate with the solid support and as long as it is sterilizable, and preferably not toxic. As an example, the holder may be produced by rapid prototyping, wherein acrylonitrile butadiene styrene is used as a material for the holder.

According to the present invention, a cell-culture dish comprising a liquid is provided below the holder. The term "liquid" as used herein is not particularly limited and relates to any liquid that might be of interest for cultivating cells or 3D cell structures. In particular, the liquid can be selected from the group consisting of water, aqueous solutions and aqueous media. Preferably, phosphate-buffered saline (PBS) is used. The liquid in the cell-culture dish provides a humid atmosphere advantageous for cultivating cells or 3D cell structures in the hanging separated homogeneous aqueous fluid microdroplets. The legs of the holder enable easy refilling of the liquid into the cell-culture dish in order to keep a stable humid atmosphere by providing sufficient space between the plate of the holder and the ground. Additionally, mixing of the hanging separated homogeneous aqueous fluid microdroplets and the liquid in the cell-culture dish can be avoided by using a holder comprising legs of sufficient height.

The patterned substrates used in the method of the present invention, as well as methods for producing the same, are preferably as described hereinafter.

In the following, the terms "hydrophilic" and "superwetting" will be used synonymously.

The hydrophobic, preferably superhydrophobic, surfaces and barriers of the patterned substrate used in the present invention result in the prevention of proliferation and migration of cells. The use of such hydrophobic, preferably superhydrophobic, barriers is an approach for controlling cell proliferation and migration. The combination of the watertight hydrophilic (superwettable) areas with the highly efficient cell-barriers represents an approach for designing e.g. cell arrays and has the potential to push the spot-density towards its theoretical maximum that is only given by the necessary amount of cells in each spot. Stringent control over all important parameters (feature shape and size, absorbable volume, thickness of the polymer film, cell-migration, transparency) and the simple and inexpensive production enables an easy adjustment to any other application where cell-growth in a predetermined spatial order on a flat substrate is needed. In view of the high spot-density, it is also possible to produce a high density of 3D cell structures such that the combination of the patterned surface and the method of producing 3D cell structures is particularly advantageous.

In general, surface properties can be classified into hydrophobic and hydrophilic surfaces depending on the value of the water contact angle (WCA). A surface having a WCA greater than 90° is referred to as hydrophobic, whereas a WCA smaller than 90° is referred to as hydrophilic. The maximum water contact angle on a smooth surface is about 120°. In practice, two types of WCA values are used: static and dynamic. Static water contact angles (qstat) are obtained by sessile drop measurements, where a drop is deposited on the surface and the value is obtained by a goniometer or a specialized software. Dynamic contact angles are non-equilibrium contact angles and are measured during the growth (advancing WCA qadv) and shrinkage (receding WCA qrec) of a water droplet. The difference between qadv and qrec is defined as contact angle hysteresis (CAH). In a preferred embodiment of the present invention, surfaces with high WCA, preferably greater than 130°, more preferably greater than 140°, most preferably greater than 150°, and/or low water CAH (less than about 30°, preferably less than about 20°, more preferably less than about 10°) are called superhydrophobic. Water droplets do not stick to such surfaces and simply roll off. In a preferred embodiment of the present invention, surfaces with both static, advancing and receding WCAs less than 20°, preferably less than 10°, more preferably less than 5°, most preferably close to or of 0° are called superhydrophilic. Water droplets are rapidly absorbed by such surfaces.

In a preferred embodiment of the present invention, the patterned substrate is a surface having hydrophilic, preferably superhydrophilic, patterns on a hydrophobic, preferably superhydrophobic, background. In a more preferred embodiment of the present invention, the patterned substrate is a surface having 588, 2187 and 4563 hydrophilic, preferably superhydrophilic, areas on a hydrophobic, preferably superhydrophobic, background per standard microscope glass slide.

Superhydrophobic and superhydrophilic materials are known in the art. Within the scope of the present invention, the layer is a porous polymer layer which exhibits hydrophobic, preferably superhydrophobic, properties and which can be comprised of a crosslinked polyvinyl monomer, wherein the polyvinyl monomer is one or more monomers selected from the group consisting of alkylene diacrylates, oligoethyleneoxide diacrylates or dimethacrylates, alkylene dimethacrylates, pentaerythritol tetraacrylate, pentaerythritol tetramethacrylate, trimethylopropane acrylate, trimethylopropane methacrylate, divinylbenzene, and divinylnaphthalene. In a preferred embodiment, the polyvinyl monomer is selected from the group consisting of ethylene dimethacrylate and divinylbenzene, whereof ethylene dimethacrylate is particularly preferred.

The hydrophobic, preferably superhydrophobic, porous polymer may further comprise a monovinyl monomer, wherein the monovinyl monomer is selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, styrene, vinylnaphthalene, vinylanthracene, and derivatives thereof, wherein the alkyl group in each of the alkyl monomers has 1-18 carbon atoms. In a preferred embodiment, the monovinyl monomer is selected from the group consisting of methyl methacrylate, butyl methacrylate, benzyl methacrylate and styrene. The above polyvinyl monomer is copolymerized with the monovinyl monomer affording the porous hydrophobic, preferably superhydrophobic, polymer layer.

According to the present invention, in the embodiment that the layer is a porous polymer layer exhibiting hydrophilic, preferably superhydrophilic, properties, the porous polymer layer can be comprised of a crosslinked polyvinyl monomer, wherein the polyvinyl monomer is one or more monomers selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate. In a preferred embodiment, the polyvinyl monomer is selected from the group consisting of ethylene dimethacrylate and methylene-bis-acrylamide.

The hydrophilic, preferably superhydrophilic, porous polymer may further comprise a monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamido-glycolic acid, [2-(methacryloyloxy)ethyl] trimethylammonium chloride, and N-[3-(dimethylamino)propyl] methacrylamide. In a preferred embodiment, the monovinyl monomer is selected from the group consisting of 2-hydroxyethyl methacrylate, decaethylene glycol methacrylate, N-isopropylacrylamide, and acrylamide. The above polyvinyl monomer is copolymerized with the monovinyl monomer affording the porous hydrophilic, preferably superhydrophilic, polymer layer.

In a particularly preferred embodiment of the present invention, the layer is a porous polymer layer comprising at least one member selected from the group consisting of porous poly(butyl methacrylate-co-ethylene dimethacrylate) (BMAcoEDMA), porous poly(methyl methacrylate-co-ethylene dimethacrylate) (MMAcoEDMA), porous poly(2-hydroxyethyl methacrylate-co-ethylene dimethyl-acrylate) (HEMAcoEDMA), porous poly(styrene-co-1,4-divinylbenzene) (STcoDVB), porous poly(2,2,3,3,3-pentafluoropropyl methacrylate-co-ethylene dimethacrylate) (PFPMAcoEDMA).

In a preferred embodiment of the patterned substrate used in the present invention, the pattern is created by modifying the surface of the layer or film using photografting or a thiol-yne reaction through a photomask. In a more preferred embodiment of the present invention, the hydrophobic as well as hydrophilic areas are created by the thiol-yne chemistry-based modification of the layer being a porous HEMA-EDMA layer functionalized with alkyne groups. In particular, a thiol-yne chemistry-based modification as described in Feng, W. et al., Surface Patterning via Thiol-Yne Click Chemistry: An Extremely Fast and Versatile Approach to Superhydrophilic-Superhydrophobic Micropatterns, Advanced Materials Interfaces 2014, 1400269, pp. 1 to 6, can be used in the present invention. In another preferred embodiment of the present invention, the hydrophilic areas are created by photografting the layer or film being a hydrophobic layer or film.

In a preferred embodiment of the present invention, the patterned substrate is a microarray which preferably exhibits an array-pattern which is created by photografting or the thiol-yne based modification of the layer or film using a photomask. The use of photochemical methods allows precisely controlling the geometry, size and distance between the spots by the design of the photomask.

According to the present invention, the layer or film itself may be hydrophilic, preferably superhydrophilic. In this embodiment, the hydrophilic, preferably superhydrophobic, barriers originate from the layer or filmmodified by photografting or preferably by thiol-yne chemistry-based modification of the layer or film. On the other hand, the layer or film itself may be hydrophilic, preferably superhydrophilic. In such an embodiment, the hydrophobic, preferably superhydrophobic, barriers are created by photografting the layer or film or by thiol-yne chemistry-based modification of the layer or film.

According to the present invention, the hydrophobic, preferably superhydrophobic, barriers are preferably created by modifying, preferably photografting or thiol-yne reacting the layer or film being a (super)hydrophilic layer or film. In a preferred embodiment of the present invention, the hydrophobic, preferably superhydrophobic, barriers are obtained by UV-initiated photografting or thiol-yne based modification of the layer or film using a compound capable to impart a hydrophobic, preferably superhydrophobic, functionality to the hydrophilic, preferably superhydrophilic, or alkyne-modified layer or film. In one embodiment of the present invention corresponding to the modification by UV-initiated photografting, the hydrophilic, preferably superhydrophilic, layer or film is photografted using a photografting mixture containing one or more mono- or poly- vinyl monomers selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, for example 2,2,3,3,3-pentafluoropropyl methacrylate, styrene, vinyl naphthalene, vinylanthracene, alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate and derivatives thereof, wherein the alkyl group in each of the alkyl monomers has 1-18 carbon atoms. Particularly preferred is a photografting mixture consisting of 15wt.% 2,2,3,3,3-pentafluoropropyl methacrylate, 1wt.% ethylene dimethacrylate in 1/3 (v./v.) mixture of water - tert-butanol containing 0.25wt.% benzophenone.

According to one embodiment of the present invention, the hydrophilic, preferably superhydrophilic, areas may be created by modifying, preferably photografting the layer or film being a hydrophobic, preferably superhydrophobic, layer or film or by thiol-yne chemistry modification of said layer or film. In the embodiment corresponding to UV-initiated photografting, imparting hydrophilic, preferably superhydrophilic, properties to the hydrophobic, preferably superhydrophobic, layer or film is accomplished using a hydrophilic compound, for example mono- or polyvinyl monomer selected from the group consisting of monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1- propanesulfonic acid, 2-acrylamido-glycolic acid, [2-(methacryloyloxy)ethyl] trimethylammonium chloride, N-[3-(dimethylamino)propyl] methacrylamide or alkylene diacrylates, olygoethyleneoxide diacrylates or dimethacrylates, alkylene dimethacrylates, pentaerythritol tetraacrylate, pentaerythritol tetramethacrylate. Preferably, said hydrophilic compound is a photografting mixture comprising [2-(methacryloyloxy)ethyl]-trimethylammonium chloride (META) or 2-acryloamido-2-methyl-1-propanesulfonic acid. According to a more preferred embodiment, the hydrophilic photografting mixture consists of 15wt.% META, and 0.25wt.% benzophenone dissolved in a 1:3 (v/v) mixture of water and tert-butanol.

According to a preferred embodiment of the present invention, the hydrophilic/hydrophobic spots/areas are created by thiol-yne chemistry-based modification of the layer or film according to the procedure described in the above-cited document Feng, W. et al., Surface Patterning via Thiol-Yne Click Chemistry: An Extremely Fast and Versatile Approach to Superhydrophilic-Superhydrophobic Micropatterns, Advanced Materials Interfaces 2014, 1400269, pp. 1 to 6.

In the thus obtained pattern, an easy and homogeneous adsorption of the spotting solution in the hydrophilic areas can be guaranteed. Each area is separated from adjacent areas with hydrophobic, preferably superhydrophobic, barriers that render the areas impermeable for all water based solutions.

In a preferred embodiment of the present invention, the hydrophilic areas of the patterned substrate are separated by the hydrophobic areas so as to form an array of hydrophilic areas surrounded by a hydrophobic surface.

In another preferred embodiment of the patterned substance according to the present invention, the hydrophilic areas are separated by the hydrophobic, preferably superhydrophobic, areas of surface of the layer or film so as to form hydrophilic, preferably superhydrophilic, channels. In a more preferred embodiment of the present invention, the patterned substance comprises hydrophilic, preferably superhydrophilic, channels on a hydrophobic, preferably superhydrophobic, background and the patterned substance is used for microfluidic applications.

In another preferred embodiment of the present invention, the hydrophilic, preferably superhydrophilic, areas which are separated by the hydrophobic, preferably superhydrophobic, barriers in the patterned substrate have a square shape. Hydrophilic, preferably superhydrophilic, areas having a square shape allow dense packing of the areas and ease the readout. Nevertheless, the patterned substrate according to the present invention is not limited to any array-format, but represents a more general approach that allows patterning and for example culturing of cells in a predesigned spatial order.

In one embodiment of the present invention, the area-density of the hydrophilic, preferably superhydrophilic, areas is increased. Preferably, the amount of hydrophilic, preferably superhydrophilic, areas is at least 9 per cm² of the patterned substrate, more preferably at least 60 per cm² of the patterned substrate, even more preferably at least 190 per cm² of the patterned substrate. In a particularly preferred embodiment of the present invention, the amount of hydrophilic, preferably superhydrophilic, areas is at least 300 per cm² of the patterned substrate.

According to an even more preferred embodiment of the present invention, the patterned substrate used in the present invention has a size which fits on a standard microtiter scaled plate (12 × 8 cm), preferably a size of about 11 × 7 cm with at least 5000, preferably 20000, more preferably 50.000 or 60000 superhydrophobic background. In another preferred embodiment of the present invention, the patterned substrate used in the present invention is a microscope glass slide with dimensions of 2.5 cm × 7.5 cm.

Preferably, in the patterned substrate used in the present invention, the overall number of spots on a single chip is significantly increased. When used as a cell microarray, minimal area of a single area in the patterned substrate according to the present invention is limited by the amount of cells needed in each area to obtain statistically valid data. In a particularly preferred embodiment of the present invention, each of the hydrophilic, preferably superhydrophilic, areas defined in (i) has a side length of 3000 µm or less, preferably 1000 µm or less, more preferably 500 µm or less, and most preferably 335 µm or less, particularly under the provision that the hydrophilic, preferably superhydrophilic, areas have a square shape. In another preferred embodiment of the present invention, the sizes of the hydrophilic, preferably superhydrophilic, areas are large enough to accommodate from 50 to 400 cells, preferably from 75 to 300 cells, more preferably 100 to 200 cells. By providing the aforementioned number of cells in one hydrophilic, preferably superhydrophilic, area 3D cell structures can be formed.

In a preferred embodiment of the present application the hydrophobic, preferably superhydrophobic, barriers have a width of 500 µm or less, preferably 200 µm or less, more preferably 100 µm or less and even more preferably 60 µm or less. A significant advantage of the patterned substrate according to the present invention is that the hydrophobic, preferably superhydrophobic, barriers completely prevent cross-contamination between the areas, despite the reduced width of the barriers.

Further, according to the present invention, the thickness of the layer or film is not limited. Regarding transparency properties or reducing manufacturing costs, a thin layer or film is preferred. Particularly preferred is a layer or film having a thickness of 45 µm or less, preferably 30 µm or less, and more preferably 15 µm or less.

The patterned substrate used in the present invention preferably further comprises (iii) at least one biologically active agent provided in at least one hydrophilic, preferably superhydrophilic, area. According to a preferred embodiment, each hydrophilic, preferably superhydrophilic, area of the patterned substrate is provided with at least one biologically active agent. In this embodiment, the hydrophobic, preferably superhydrophobic, barriers not only prevent cross-contamination of any aqueous liquids between the areas, but also prevent proliferation and migration of cells between hydrophilic areas. That is, hydrophobic, preferably superhydrophobic, barriers between the hydrophilic, preferably superhydrophilic, areas of the patterned substrate according to the present invention stop cell proliferation and migration from one area to another. With this technique, a patterned substrate can be designed with an area-density that is not achievable with common methods.

The very good wetting property of the surface of the layer or film inside hydrophilic areas guarantees an easy and homogeneous spreading of the solutions inside the areas independent of their geometry. In addition, the hydrophobic, preferably superhydrophobic, barriers prevent cells from migrating between the adjacent areas. Thus, the serious problem of cell-migration between the areas can be avoided using the patterned substrate as described herein.

The patterned substrate coated with a layer or film, wherein the layer is a porous polymer layer, used in the present invention can be manufactured by a method, comprising the steps of:
(a) providing a polymerization mixture between two solid supports;
(b) polymerizing the polymerization mixture to form a porous polymer layer;
(c) removing one of the two solid supports; and
(d) modifying the surface of the porous polymer layer, thereby creating a pattern of hydrophilic areas surrounded by hydrophobic areas.

The solid support may be of any material known in the art which is solid, preferably at a temperature within the range of -30°C to 130°C, more preferably within the range of 0°C to 40°C, more preferably at room temperature. The solid support can be selected from the group consisting of glass, metal, such as a stainless steel plate, or an aluminum foil, plastic, concrete, wood, and masonry. Preferably, the solid support is transparent, wherein a glass solid support is particularly preferred. Such a glass solid support may be activated and/or modified prior to use.

The size of the solid support is not particularly limited. According to a preferred embodiment of the present invention, the solid support is of standardized size. Standardized solid supports can be fixed in commonly used screening microscopes.

In order to achieve covalent attachment of the porous polymer layer, the glass surface is preferably first activated, and then functionalized with an anchor-group. For example, cleaned glass plates may be immersed in 1 M NaOH for one hour and afterwards washed with deionized water followed by immersing them in 1 M HCl for 30 min, washing with deionized water and drying with a nitrogen gun. Further, the glass solid support may be modified by for example dropping several drops of a solution containing 20% 3-(trimethoxysilyl)propyl methacrylate in ethanol (adjusted to pH = 5 with acetic acid) on an activated glass plate, covering the glass plate with another activated glass plate, thereby ensuring that no bubbles are trapped between the two glass plates, reapplying said solution after 30 minutes and after another 30 minutes washing the glass plates with acetone and drying with a nitrogen gun.

The glass solid supports may be made inert for example by fluorination using for example tridecafluor-(1,1,2,2)-tetrahydrooctyltrichlorosilane placed in a vacuumed desiccator together with the glass solid support for 2h.

In step (a) of the method for the manufacture of a patterned substrate, a polymerization mixture is provided between two solid supports. The solid supports may be made of the same material, or of different materials. In particular, the aforementioned materials suitable for the patterned substrate according to the present invention are preferred. Preferably, the solid supports are made of a glass material, of which one or both may be modified and/or activated prior to use, such as a glass modified with anchor groups. Preferably, the solid support is a solid support as defined herein.

In step (b) of the method for the manufacture of a patterned substrate, polymerization is carried out in the mold between the two solid supports. The polymerization may involve a free radical polymerization of a polyvinyl monomer and a monovinyl monomer in the presence of porogenic solvents. The free radical initiation is preferably done either thermally or by irradiation with UV- or visible light or γ-irradiation. When polymerization is carried out by thermal initiation, the thermal initiator is generally a peroxide, a hydroperoxide, or an azo-compound selected from the group consisting of benzoyl peroxide, potassium peroxodisulfate, ammonium peroxodisulfate, t-butyl hydroperoxide, 2,2'-azobisiobutyronitrile (AIBN), and azobisisocyanobutyric acid, and the thermally induced polymerization is performed by heating the polymerization mixture to temperatures between 30°C and 120°C. Polymerization can also be achieved using photoinitiators including, but not limited to, benzophenone, 2,2-dimethoxy-2-phenylaceto-phenone, dimethoxyacetophenone, xanthone, thioxanthone, camphorquinone their derivatives, and mixtures thereof.

The polymerizing mixture is not limited, as long as said polymerizing mixture affords a porous polymer layer having hydrophobic, preferably superhydrophobic, or hydrophilic, preferably superhydrophilic, properties. Depending on the aimed surface property of the porous polymer, i.e. hydrophilicity or hydrophobicity, the respective monomers are selected accordingly.

Preferably, the porous polymer layer possesses bulk hydrophilicity and is prepared by radical polymerization of a mixture containing a polyvinyl crosslinker and monovinyl monomers in the presence of an inert porogen.

The porogen (i.e. liquids that cause formation of the porous structure of the polymer) used to prepare the porous polymer layer may be selected from a variety of different types of compounds. For example, suitable liquid porogens include aliphatic hydrocarbons, aromatic hydrocarbons, esters, amides, alcohols, ketones, ethers, solutions of soluble polymers, and mixtures thereof. For preparation of hydrophilic, preferably superhydrophilic, materials, water may also be used. The porogen is generally present in the polymerization mixture in an amount of from about 40 to 90 vol%, more preferably from about 50 to 80 vol%. In a preferred embodiment, the porogen is a mixture of 1- decanol and cyclohexanol.

Preferably, the polyvinyl crosslinker is selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1 - 4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate.

Preferably, the monovinyl monomers are selected from the group consisting of alkyl vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1 - 4 carbon atoms, hydroxyalkyl acrylates and acrylamides, hydroxymethacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamidoglycolic acid, [2-(methacrylooxy)ethyl] trimethylammonium chloride, and N-[3-(dimethylamino)propyl]methacrylmide.

Preferably, the inert porogen is selected from the group consisting of aliphatic hydrocarbons, aromatic hydrocarbons, esters, amides, alcohols, ketones, ethers, solutions of soluble polymer, water, and mixtures thereof.

Preferably, the porous polymer layer comprises at least one member selected from the group consisting of porous poly(butyl methacrylate-co-ethylene dimethacrylate) (BMAcoEDMA), porous poly(methyl methacrylate-co-ethylene dimethacrylate) (MMAcoEDMA), porous poly(2-hydroxyethyl methacrylate-co-ethylene dimethyl-acrylate) (HEMAcoEDMA), porous poly(styrene-co-1,4-divinylbenzene) (STcoDVB), porous poly(2,2,3,3,3-pentafluoropropyl methacrylate-co-ethylene dimethacrylate) (PFPMAcoEDMA).

More preferably, the hydrophilic porous polymer layer is made of poly(2-hydroxyethyl methacrylate-co-ethylene dimethacrylate) synthesized by copolymerization of 2-hydroxyethyl methacrylate (HEMA) and ethylene dimethacrylate (EDMA) in the presence of porogens (as shown for example in Fig. 1).

Preferably, the porous polymer layer is made of poly(butyl methacrylate-co-ethylene dimethyl-acrylate) (BMAcoEDMA). The porous polymer layer of BMAcoEDMA is synthesized by copolymerization of butyl methacrylate (BMA) and ethylene dimethacrylate (EDMA) in the presence of porogens.

In the embodiment that the porous polymer layer should exhibit hydrophobic, preferably superhydrophobic, properties, the polyvinyl monomer is one or more monomers selected from the group consisting of alkylene diacrylates, alkylene dimethacrylates, penta-erythritol tetraacrylate, pentaerythritol tetramethacrylate, trimethylopropane acrylate, trimethylopropane methacrylate, divinylbenzene, and divinyl-naphthalene. Preferably, the polyvinyl monomer is selected from the group consisting of ethylene dimethacrylate and divinylbenzene, whereof ethylene dimethacrylate is particularly preferred.

Further, the hydrophobic, preferably superhydrophobic, porous polymer further comprises a monovinyl monomer, wherein the monovinyl monomer is selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, styrene, vinylnaphthalene, vinylanthracene, and derivatives thereof, wherein the alkyl group in each of the alkyl monomers has 1-18 carbon atoms. Preferably, the monovinyl monomer is selected from the group consisting of methyl methacrylate, butyl methacrylate, benzyl methacrylate and styrene. The above polyvinyl monomer is copolymerized with the monovinyl monomer in the presence of a porogenic solvent affording the porous hydrophobic, preferably superhydrophobic, polymer layer.

In the embodiment that the porous polymer layer should exhibit hydrophilic, preferably superhydrophilic, properties, the porous polymer layer can be comprised of a crosslinked polyvinyl monomer, wherein the polyvinyl monomer is one or more monomers selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate. Preferably, the polyvinyl monomer is selected from the group consisting of ethylene dimethacrylate and methylene-bis-acrylamide.

The hydrophilic, preferably superhydrophilic, porous polymer further comprises a monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamido-glycolic acid, [2-(methacryloyloxy)ethyl] trimethylammonium chloride, and N-[3-(dimethyl-amino)propyl] methacrylamide. Preferably, the monovinyl monomer is selected from the group consisting of 2-hydroxyethyl methacrylate, decaethylene glycol methacrylate, N-isopropylacrylamide, and acrylamide. The above polyvinyl monomer is copolymerized with the monovinyl monomer in the presence of a porogenic solvent affording the porous hydrophilic, preferably superhydrophilic, polymer layer.

The ratio of polyvinyl monomer to monovinyl monomer is not limited as long as the obtained porous polymer exhibits hydrophobic, preferably superhydrophobic, properties or hydrophilic, preferably superhydrophilic, properties. In a preferred embodiment, the polyvinyl monomer content is within the range of 5 to 30wt.%, preferably 10 to 25wt.%, more preferably 15 to 20wt.%, based on the total amount of the polymerization mixture. The monovinyl monomer content is preferably within the range of 10 to 50wt.%, preferably 15 to 45wt.%, more preferably 20 to 40wt.%, based on the total amount of the polymerization mixture.

Particularly preferred polymerization mixtures include:
(i) poly(2-hydroxyethyl methacrylate-co-ethylene dimethacrylate) (HEMA-co-EDMA): 2-hydroxyethyl methacrylate (HEMA) (24wt.%), ethylene dimethacrylate (EDMA) (16wt.%), 1-decanol (12wt.%), Cyclohexanol (48wt.%) and 2,2-dimethoxy-2-phenylaceto-phenone (DMPAP) (1wt.% with respect to monomers),
(ii) poly(2-hydroxyethyl methacrylate-co-ethylene dimethacrylate): 2-hydroxy-ethyl methacrylate (24wt.%), ethylene dimethacrylate (16wt.%), 1-decanol (40wt.%), cyclohexanol (20wt.%) and 2,2-dimethoxy-2-phenylacetophenone (1wt.% with respect to monomers), and
(iii) poly(butyl methacrylate-co-ethylene dimethacrylate) (BMAcoEDMA): butyl methacrylate (BMA) (24wt.%), ethylene dimethacrylate (16wt.%), 1-decanol (40wt.%), cyclohexanol (20wt.%) and 2,2-dimethoxy-2-phenylacetophenone (1wt.% with respect to monomers).

After completion of the polymerization, one of the two substrates is removed in step (c).

In step (d) of the method for the manufacture of a patterned substrate, the surface of the porous polymer layer is modified, thereby creating a pattern of hydrophilic areas surrounded by hydrophobic areas. Preferably, the modification is carried out as described herein, most preferably by photografting including the various embodiments for photografting as described herein or by thiol-yne based modification as described in Feng, W. etal., Surface Patterning via Thiol-Yne Click Chemistry: An Extremely Fast and Versatile Approach to Super hydrophilic-Super hydrophobic Micropatterns, Advanced Materials Interfaces 2014, 1400269, pp. 1 to 6.

In case that photografting is applied, the hydrophobic pattern is preferably produced by photografting of the hydrophilic porous polymer layer with hydrophobic mono- or poly-vinyl monomers or mixtures thereof.

Alternatively, the hydrophobic pattern is preferably produced by photografting of the hydrophilic porous polymer layer with hydrophobic mono- or poly-vinyl monomers or mixtures thereof and the monovinyl monomers are selected from the group consisting of alkyl acrylates, alkyl methacrylates, aryl acrylates, aryl methacrylates, aryl alkyl acrylates, aryl alkyl methacrylates, fluorinated alkyl acrylates, fully or partially fluorinated alkyl methacrylates, for example 2,2,3,3,3-pentafluoropropyl methacrylate, styrene, vinyl naphthalene or vinylanthracene and derivatives thereof, wherein the alkyl group in each of the alkyl monomers has 1-20 carbon atoms.

As another alternative, the hydrophobic pattern may be produced by photografting of the hydrophilic porous polymer layer with hydrophobic mono- or poly-vinyl monomers or mixtures thereof and the polyvinyl monomers are selected from the group consisting of alkylene diacrylates, alkylene dimethacrylates, pentaerythritol tetraacrylates, pentaerythritol tetramethacrylates, trimethylopropane acrylates, trimethylopropane methacrylates, divinylbenzenes and divinylnaphthalenes.

According to a preferred embodiment of the method employing photografting, the modification of the surface of the porous layer in step (d) of the method for the manufacture of a patterned substrate is carried out by photografting the surface of the porous polymer layer using a photomask, wherein a photografting mixture is provided on the surface of the porous polymer layer. Said photografting creates a pattern of hydrophobic, preferably superhydrophobic, barriers separating hydrophilic, preferably superhydrophilic, areas. As described earlier, the photomask may be applied so as to create the pattern, or to create the areas. Depending on the nature of the porous polymer layer (hydrophilic, preferably superhydrophilic, or hydrophobic, preferably superhydrophobic), the photografting mixture applied to the porous polymer layer may induce either hydrophobic, preferably superhydrophobic, or hydrophilic, preferably superhydrophilic, regions on the porous polymer layer.

The polymerization of the polymerizing mixture may afford a hydrophilic, preferably superhydrophilic, polymer layer which is subsequently photografted with a compound capable to impart superhydrophobicity to the porous polymer layer using a photomask.

In this embodiment, the hydrophilic, preferably superhydrophilic, areas originate from the regions of the porous polymer layer which have not been photografted. In addition, the regions which are photografted are transformed into hydrophobic, preferably superhydrophobic, barriers separating the hydrophilic, preferably superhydrophilic, areas.

According to one embodiment of the present invention, the porous polymer layer is a HEMAcoEDMA polymer layer, and the hydrophobic, preferably superhydrophobic, barriers are created by photografting the HEMAcoEDMA surface with a photografting mixture. Preferred is a photografting mixture containing 2-,2-,3-,3-,3-pentafluoropropyl methacrylate (PFPMA). In a more preferred embodiment of the present invention, the hydrophilic porous polymer surface comprises poly(2-hydroxyethyl methacrylate-co-ethylene dimethyl-acrylate) and the photografted hydrophobic monomer is 2,2,3,3,3-pentafluoropropyl methacrylate.

The photografting mixture may consists of 15wt.% of 2,2,3,3,3-pentafluoropropyl methacrylate and 1wt.% of ethylene dimethacrylate in 1/3 (v./v.) mixture of water- tert-butanol containing 0.25wt.% benzophenone.

Preferably, the hydrophilic porous polymer surface comprises HEMAcoEDMA and the photografted hydrophobic monomer is PFPMA. More preferably, a 12.5 µm-thin hydrophilic porous HEMAcoEDMA layer is patterned with PFPMA to form an array of square hydrophilic HEMAcoEDMA areas separated by hydrophobic areas.

The preparation of a patterned substrate ready for further applications such as printing biomolecules takes preferably about one hour.

The method for the manufacture of a patterned substrate described herein preferably further comprises the step (e) providing at least one biologically active agent in at least one hydrophilic, preferably superhydrophilic, area, more preferably in each of the hydrophilic, preferably superhydrophilic, areas.

In particular embodiments, the patterned substrates used in the method of the present invention, as well as methods for producing the same, are as described in European patent applications 10 015 106.7 and/or 12 005 164.4 and/or as described in Geyer, F. L. et al., Superhydrophobic-Superhydrophilic Micropatterning: Towards Genome-on-a-Chip Cell Microarrays, Angewandte Chemie International Edition 2011, 50, pp. 8424-8427 and/or Feng, W. et al., Surface Patterning via Thiol-Yne Click Chemistry: An Extremely Fast and Versatile Approach to Superhydrophilic-Superhydrophobic Micropatterns, Advanced Materials Interfaces 2014, 1400269, pp. 1 to 6.

Further, a general method for high-throughput screening of 3D cell structures comprises providing 3D cell structures produced by the aforementioned method; and high-throughput screening of the 3D cell structures. This can, for example, be advantageously combined with the droplet-array sandwich chip for high-throughput screening which is disclosed in Popova, A. A. et al., Droplet-Array (DA) Sandwich Chip: A Versatile Platform for High-Throughput Cell Screening Based on Superhydrophobic-Superhydrophilic Micropatterning, Advanced Materials 2015, 27, pp. 5217-5222. Said droplet-array sandwich chip enables a precise distribution of the desired compound for HT screening at the same time to many spots. In combination with the easy one-step application of the aqueous fluid to the multitude of the hydrophilic areas used in the above method, said chip and the patterned substrate provide a good basis for HT screening of 3D cell structures. In addition, since the volume of the aqueous fluid needed in the method is small, costs of HT screening can be considerably reduced.

According to the present invention, provided that analyses and processing of the 3D cell structures comprised in the microdroplets is not desired, it is also possible to harvest the 3D cell structures at any time by bringing the hanging separated homogenous aqueous fluid microdroplets comprising the 3D cell structures into contact with a liquid contained for example in the cell-culture dish provided below the holder. Upon contact of the microdroplets and the liquid, the 3D cell structures are passed over to the liquid and sink. The 3D cell structures may be collected in this way for further analysis and characterization for example for histological analysis or colorimetric or luminescence viability assays, as well as for fabrication of complex systems combining 3D cell structures of different origin in one culture flask.

In addition, the present invention relates to a hanging droplet device comprising an array of hanging separated homogenous aqueous fluid microdroplets of a desired shape and size in a desired spatial pattern, wherein the hanging separated homogenous aqueous fluid microdroplets comprise 3D cell structures, hanging from a patterned substrate with a solid support coated with a layer or film, wherein the patterned substrate comprises
(i) hydrophilic areas having the desired shape and size; surrounded by
(ii) hydrophobic areas separating the hydrophilic areas into the desired spatial pattern, and

wherein the patterned substrate faces down,
wherein the patterned substrate with the solid support is provided on a holder comprising a plate having an opening which is adjusted to hold the patterned substrate with the solid support,
wherein a cell-culture dish comprising a liquid is provided below the holder,
wherein the volume of one hanging separated homogenous aqueous fluid microdroplet is in the range from 5 nL to 100 nL, and
wherein the holder is a table comprising a tabletop with an opening which is adjusted to hold the turned patterned substrate with the solid support and comprises legs which extend from the surface of the plate facing downwards to the ground.

The hanging droplet device of the present invention represents a state of the array of hanging separated homogeneous aqueous fluid microdroplets of a desired shape and size in a desired spatial pattern, hanging from the patterned substrate with the solid support of the aforementioned method of producing 3D cell structures after performing step (3). In particular, the hanging droplet device describes a state of the patterned substrate in which said patterned substrate with the solid support is turned upside down (*i.e.* as a result of step (2) of the method of producing 3D cell structures) such that the patterned substrate faces down.

In the hanging droplet device according to the present invention, the patterned substrate with the solid support is provided on a holder comprising a plate having an opening which is adjusted to hold the patterned substrate with the solid support. The holder on which the patterned substrate is provided corresponds to the holder used in the method of producing 3D cell structures according to the present invention.

Further, the present invention relates to a use of the aforementioned hanging droplet device for high-throughput screening of the 3D cell structures.

According to another embodiment, the present invention relates to a use of the above-mentioned hanging droplet device for cultivating 3D cell structures.

In the present application, a facile method for producing 3D cell structures is described. It has been shown that 3D cell structures can be trapped in completely isolated microdroplets which can be used in a variety of screening applications.

The present invention solves the problem of providing 3D cell structures by providing in a first step the forming of arrays of separated droplets comprising cells in a desired pattern or shape in one easy step using hydrophilic-hydrophobic patterned surfaces. To form separated, homogeneous droplets, the patterned surface can simply be dipped into a solution or a droplet is rolled along the surface. The droplets formed on the surface are due to the contrast in wettability of the hydrophilic-hydrophobic pattern. This invention allows thousands (or more) spots to be filled at once without the use of expensive liquid handling devices such as contact printers, pipetting robots, acoustic droplet ejection devices, etc. Depending on the cell concentration used in the aqueous fluid, the cell density per spot can be easily adjusted. As a consequence, also the diameter of a 3D cell structure resulting from the agglomeration of the cells can be influenced in a simple way. Therefore, the size of the 3D cell structure cannot only be adjusted by changing the size of the hydrophilic spots, but also by changing the cell concentration in the aqueous fluid. Furthermore, a high density of 3D cell structures may be produced according to the present invention by the advantageous combination of the patterned surface, which provides a high spot density, and the method of producing 3D cell structures.

This invention solves the problem of providing 3D cell structures which can be further used for high-throughput screening applications. This invention eliminates the need for physical barriers to separate samples in different spots such as when using microtiter plates and allows high-density arrays to be created. For use in chemical or drug screening applications, various compounds can be added into individual aqueous fluid microdroplets formed on the patterned substrate. The present invention allows the user to create droplets of various shapes and sizes simply by forming a different hydrophilic-hydrophobic pattern.

The present invention provides the formation of hundreds of 3D cell structures of about the same size on a Droplet-Microarray, optionally followed by pooling said structures from the Droplet-Microarray into one culturing flask for further applications such as screenings or co-culturing 3D cell structures formed form different cell types.

Further, by using either cancer cell lines or primary tumor cells, tumor spheroids can be formed. On said tumor spheroids miniaturized screenings of anti-cancer drugs can be performed.

Moreover, the present invention provides miniaturized toxicity testing on spheroids formed from different cell types representing different organs such as liver, heart or kidney. For liver toxicity testing, for example, primary hepatocytes and other liver and immune cells can be used to form spheroids representing *in vivo* tumor environment. Instead of primary hepatocytes, the human liver hepatocellular cell line HepG2 can also be used.

In case that stem cells are used in the present invention, embryonic bodies can be formed with the proviso that the embryonic bodies are not human embryonic bodies and no human embryo is destroyed in the method. Such embryonic bodies can be used for screening applications to screen for example for substances that induce differentiation or keep the pluripotency, as well as for toxicity screens.

According to the present invention, miniaturized screenings involving transfection of spheroids can be carried out. For example screenings of an siRNA library on tumor spheroids can be performed for developing an anti-cancer siRNA therapy.

If several cell types are co-cultured, organoid-like structures can be produced by the present invention. By addition of different cell types at different time points it is possible to ensure formation of a desired structure. Such organoid-like structures can be used for toxicity screenings as well as for drug development.

The figures show:
Figure 1: Preparation of an array of microdroplets
   a) A patterned substrate in the dry state. A superhydrophilic, porous polymer layer is grafted with superhydrophobic moieties. b) When the hydrophobicity-patterned substrate is immersed in water, the hydrophilic areas become easily wetted while the hydrophobic areas remain in a dry Cassie-Baxter state. c) When slowly pulled out of water, only the hydrophilic areas remain filled with water and distinct droplets are formed. d) A homogeneous array of microdroplets is obtained. e) Spreading a droplet along the superhydrophilic-superhydrophobic patterned surface (1 mm diameter circles, 0.5 mm spot-to-spot spacing) forms droplets only in the hydrophilic spots.
Figure 2: Images of various arrays of microdroplets
   a) Images of arrays of microdroplets formed via the method disclosed in EP 2 684 601 A1 on superhydrophilic-superhydrophobic micropatterns with different geometries which can also be used in the present invention. b) A wetted microarray becomes transparent and displays a lens effect, showing the underlying paper printed with a logo. c) Fluorescent microscope image showing fluorescent MTly-CMV-eGFP-neo cells cultured in individual microdroplets on the array.
Figure 3: Deposition of compounds
   Deposition of a methyl green dye solution on a microarray is shown. a) Schematic representation of the droplet deposition method. The substrate is immersed in an aqueous solution, similar as described in Figure 1. b) Images of microdroplets formed from a methyl green solution (left) and then dried (right).
Figure 4: Holder
   Preferred embodiment of the holder used in the present invention. According to this embodiment, the holder is a table comprising six legs, one on each corner and additionally one in the middle of each long side, and a plate having an opening which can hold the turned patterned substrate with the solid support. Exemplary values for the size of the features of the holder are shown.

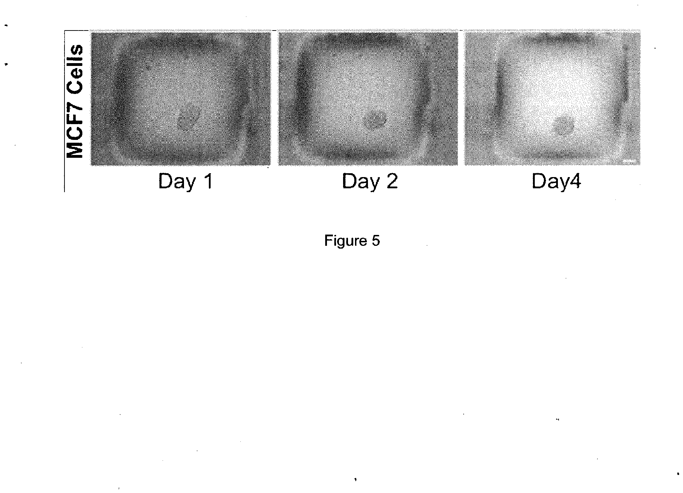

Figure 5: Example of spheroids produced according to the present invention
   Spheroids formed from human breast cancer cell line MCF7. MCF7 cells were harvested and resuspended in seeding medium containing DMEM+15%FCS+1%PenStrep. Cells were counted and cell concentration was set to 50×10⁴ cells/mL. 1.5 ml of the cell solution was pipetted onto the substrate, building one droplet that covered the whole field of an array of superhydrophilic spots. The droplet remained on the patterned surface for 60 seconds and was removed by tilting of the slide, which resulted in spontaneous formation of droplets on superhydrophilic areas containing cells. Afterwards the pattern was placed on the holder in upside down position. The holder itself together with the Droplet-Microarray was placed into a humidified cell culture dish and moved to the cell culture incubator, where it was covered with wetted tissues to prevent evaporation.

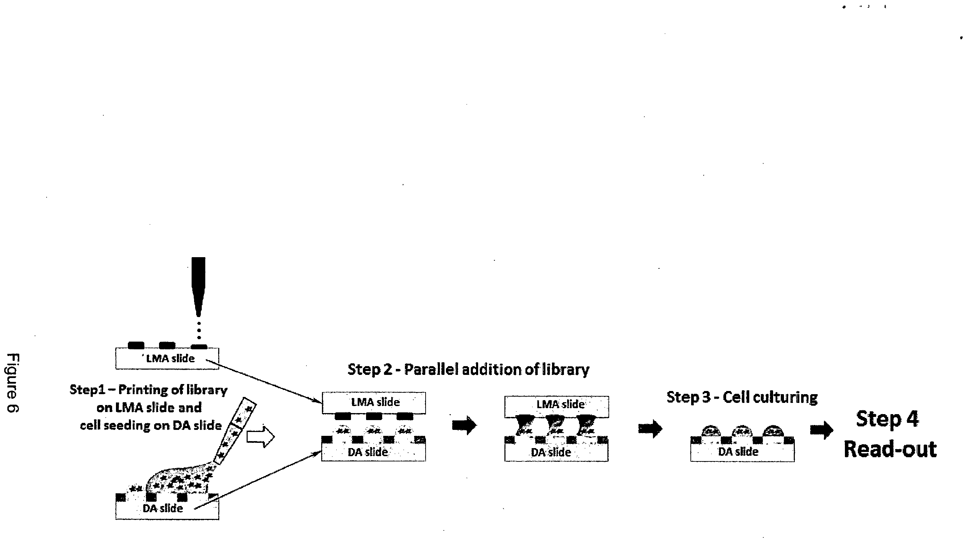

Figure 6: Schematic of a workflow of cell-based screening using Droplet-Microarray Sandwich Chip.
   LMA slide is prepared by printing of substances of interest on a glass slide (Step 1); DA slide is prepared by seeding cells using the effect of discontinuous dewetting (Step 1). For parallel addition of library into individual dropelts LMA slide is aligned and sandwiched with DA slide containing cells (Step 2). After substances are transferred into droplets LMA slide is removed and DA slide is placed into cell culturing incubator (Step 3) following by read-out (step 4).

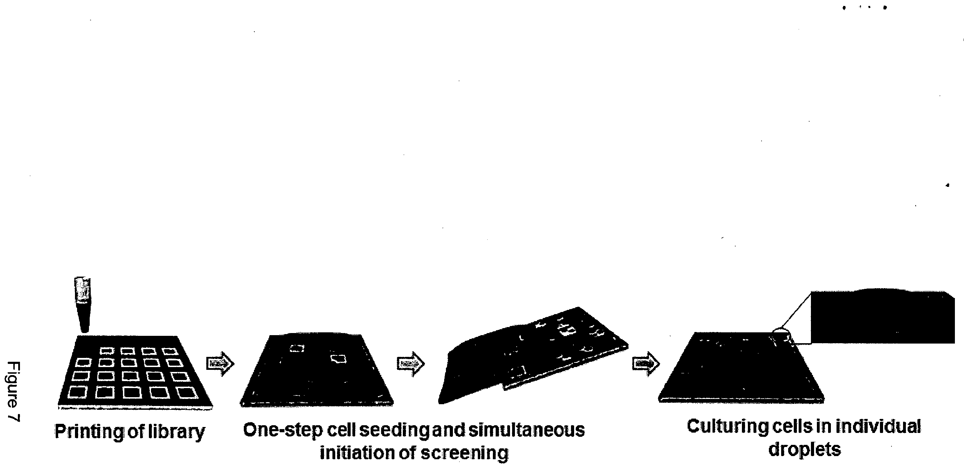

Figure 7: Schematic diagram of the workflow of reverse cell screening using DMA platform.
   Schematic diagram of the workflow of reverse cell screening using DMA platform. Compounds of interest are preprinted directly into supehydrophilic spots and cells are seeded onto preprinted Droplet-Microarray slides.

The present invention will now be further illustrated in the following examples without being limited thereto.

### Examples

### Experimental procedures:

### General.

All polymerizations and photografting were carried out on an OAI Model 30 deep-UV collimated light source (San Jose, CA, USA) fitted with an USHIO 500 W Hg-xenon lamp (Japan). Irradiation intensity was calibrated to 12 mW/cm² (5.10 mW/cm² after cover glass slide, 4.77 mW/cm² after cover glass slide and photomask) using an OAI 360 UV power meter with a 260 nm probe head. Schott (Germany) Nexterion Glass B UV transparent glass plates were used as substrates for polymer layers. Monomers were purchased from Sigma-Aldrich. Biochemicals were purchased from Invitrogen. Cell lines were provided by the Institute of Toxicology and Genetics at Karlsruhe Institute of Technology.

### Array preparation - Glass Surface Modification.

To achieve covalent attachment of the polymer layer, the glass surfaces are first activated and then functionalized with an anchor group for methacrylates.

Activation of glass slides: Clean glass slides are immersed in 1 M NaOH for 1 h and afterwards washed with deionized water, and then immersed in 1 M HCl for 30 min, washed with deionized water, and dried with a nitrogen gun.

Modification of glass slides: Several drops of a solution containing 20 %vol. 3-(trimethoxysilyl)propyl methacrylate in ethanol, adjusted to pH 5 with acetic acid, are dropped on an activated glass slide. The plate is covered with another activated glass slide. No bubbles should be trapped between the two slides. The solution is reapplied after 30 min. After another 30 min, the slides are washed with acetone and dried with a nitrogen gun.

Fluorination of glass slides: An activated glass slide is placed in a vacuumed desiccator together with a vial containing several drops of tridecafluoro-(1,1,2,2)-tetrahydrooctyltrichlorosilane overnight.

### Array preparation - Polymerization Procedure.

Polymerization: Small amounts of silica beads (Cospheric LLC) with defined size (1.8 µm; 3.62 µm, 4.08 µm or 7.75 µM), are placed at the edges of a fluorinated glass slide, the polymerization mixture is pipetted on this glass and a modified glass slide is placed on top of it. Then the slides are irradiated for 15 min at 12 mW/cm² with a 260 nm UV light. The mold is then carefully opened using a scalpel.

An inert, fluorinated glass slide is used as the bottom plate. The inability of covalent attachment of the growing polymer to the fluorinated glass slide allows for the whole polymer to stick to the top, modified glass slide during the separation process. The fluorinated glass slide can be reused several times. The resulting nonporous superficial layer can be easily removed by applying and rapidly removing adhesive film (Tesa tape) immediately after separating the plates while the layer is still wetted with porogen. A homogeneous, porous surface is formed. The plate is washed extensively with methanol and kept in methanol for several hours to remove unreacted monomers and porogens.

Polymerization mixture: 2-hydroxyethyl methacrylate (24 wt %), ethylene dimethacrylate (16 wt %), 1-decanol (12 wt %), cyclohexanol (48 wt %), and 2,2-dimethoxy-2-phenylacetophenone (1 wt % with respect to monomers).

### Modification via photografting or thiol-yne reaction:

Photografting: The polymer layer is wetted with the photografting mixture and covered with a fluorinated glass slide. A photomask is placed on top and it is irradiated for 30 min with 12 mW/cm² 260 nm UV light. The obtained pattern is washed extensively with methanol and stored in methanol for several hours to remove excess monomer and porogen, and for sterilization before cell culture.

Photografting mixture: 2,2,3,3,3-pentafluoropropyl methacrylate (20 wt %), ethylene dimethacrylate (1.3 wt %), 1:3 (v/v) mixture of water:tert-butanol (78 wt %), benzophenone as the initiator (0.33 wt %).

### Thiol-yne reaction:

### Materials:

2-Hydroxyethyl methacrylate (HEMA) and ethylene dimethacrylate (EDMA) were purchased from Sigma-Aldrich (Germany) and purified using a short column filled with basic aluminum oxide to get rid of the inhibitors. The food dyes used in Figure 2.11 were obtained from August Thomsen Corp. (USA). All the other chemicals were purchased from Sigma-Aldrich (Germany) and used without further purification. HeLa-GFP cells were purchased from Biocat (Germany). The GFP-peptide containing thiol group (fluorescein-β-Ala-GGGGC) was obtained from Dr. Cornelia Lee-Thedieck at the Institute of Functional Interfaces at Karlsruhe Institute of Technology. Schott (Germany) Nexterion Glass B UV transparent glass plates were used as substrates for polymer layers. The polymerizations and photografting were carried out on an OAI Model 30 deep-UV collimated light source (San Jose, CA) fitted with an USHIO 500 W Hg-xenon lamp (Japan).

### Preparation of 12.5 µm-thin porous HEMA-EDMA films:

Here a recently published procedure developed in our group to make nanoporous HEMA-EDMA polymer layers is employed. Briefly, two 12.5 µm-thin strips of Teflon film (American Durafilm Co.) were placed at the edges of one 3-(trimethoxysilyl)propyl methacrylate modified glass-plate (25×75×1 mm, width×lengthx×hickness) and one fluorinated glass slide was clamped on top of it. 70 µL of polymerization mixture of HEMA (24 wt%), ethylene dimethacrylate (EDMA) (16 wt%), 1-decanol (12 wt%), cyclohexanol (48 wt%) and 2,2-dimethoxy-2-phenylacetophenone (DMPAP) (photoinitiator, 1 wt% with respect to monomers) was injected in the mold between the glass slides and irradiated for 15 min with 12.0 mW·cm⁻² 260 nm UV-light. The mold was then carefully opened using a scalpel. The resulting non-porous superficial surface was removed by applying and rapidly removing adhesive film ("Scotch tape") after separating the plates while the layer was still wetted. A homogeneous porous surface was formed. The plate was washed extensively with ethanol and kept in ethanol for some minutes.

### Preparation of alkyne modified HEMA-EDMA:

Two glass plates coated with a HEMA-EDMA layer were immersed into 50 mL of dichloromethane solution containing 4-pentynoic acid (111.6 mg, 1.14 mmol) and catalyst 4-(dimethylamino)pyridine (DMAP) (56 mg, 0.46 mmol). Then, the coupling reagent N,N'-diisopropylcarbodiimide (DIC) (176.5 µL, 1.14 mmol) was added to the solution cooled to about 0°C, followed by stirring the solution at RT for 4 h. The plates were then washed extensively with acetone and kept in ethanol for several minutes, followed by drying.

### Thiol-yne "click" reactions on the porous polymer layer:

According to the solubility of a thiol, 1-dodecanethiol (5 vol%) and 1H,1H,2H,2H-perfluorodecanethiol (5 vol%) were dissolved in acetone, while 2-mercaptoethanol (15 vol%), cysteamine hydrochloride (15 wt%), and 3-mercaptopropionic acid (15 vol%) were dissolved in ethanol. These thiol solutions were not degassed prior to use.

The alkyne polymer layer was wetted with the respective thiol solution and covered with a fluorinated quartz slide (25×75×1 mm, width×length×thickness). All of the fabrications of superhydrophobic or superhydrophilic layers using thiol-yne reactions were performed by UV irradiation (12.0 mW·cm⁻², 260 nm) without photoinitiator under ambient laboratory conditions. After the reactions, the samples were washed extensively with acetone and dried with a nitrogen gun.

To study the kinetics of the thiol-yne reactions on the alkyne polymer layer, polymer layers were wetted with a respective thiol solution, covered with a fluorinated quartz slide and irradiated with UV light for different times. Time exposures of UV light were controlled by a time-controller (OAI 150 Exposure Timer). The minimum exposure time is 0.1 s. After the reaction, the samples were washed extensively with acetone and dried with a nitrogen gun. Then static WCAs of the polymer surfaces were measured.

### Preparation of superhydrophobic-superhydrophilic micropatterns via thiol-yne click photopatterning:

A typical example for the preparation of superhydrophobic-superhydrophilic micropatterns using subsequent thiol-yne reactions is presented. First, the alkyne porous layer was wetted with acetone solution containing 5 vol% 1H,1H,2H,2H-perfluorodecanethiol, covered by a fluorinated quartz slide, and irradiated by UV light through a photomask for 15 s. After removing the photomask, washing with acetone and drying, the polymer layer was wetted with an ethanol solution containing 15 wt% cysteamine hydrochloride and irradiated by UV light for another 15 s. Finally, the plate was washed extensively with acetone and dried with a nitrogen gun.

After the first reaction, the exposed fluoro-surface showed superhydrophobicity with θₛₜ, θ_{adv} and θ_{rec} as high as 170°, 173°, and 164°, respectively, while the θₛₜ of unexposed alkyne functionalized areas were maintained at 124°. After the second reaction with cysteamine, the θₛₜ on the alkyne areas reduced to 4.4°. As for the fluorinated areas, the receding WCA decreased by only 2°, while the advancing WCA did not change at all and the surface maintained superhydrophobicity, confirming almost completeness of the reaction of the first step.

### Preparation of inverse patterns:

Patterns with superhydrophobic spots and superhydrophilic barriers can be created by switching the order of the thiols used for functionalization and using the same method and the same photomask.

### Using the thiol-yne patterning method for creating peptide patterns:

The alkyne HEMA-EDMA porous layer was first wetted with a 9/1 water/ethanol solution. Then, the plate was washed extensively with pure water to replace the ethanol-water solution with water. Excess of water was removed from the surface and 10 µL of the aqueous peptide solution (fluorescein-β-Ala-GGGGC, 0.25 mg/mL) was dropped on the surface. The substrate was covered with a fluorinated quartz slide and a photomask. The polymer layer was then irradiated with UV light through a photomask for 15 s (6 mW/cm², 260 nm). Then, the plate was washed extensively with ethanol and dried with a nitrogen gun.

### Example 1:

### Formation of Droplet-Microarrays

To make an array of superhydrophilic spots on a superhydrophobic surface using photografting, a 12.5 µm thin, superhydrophilic film of nanoporous poly(hydroxyethyl methacrylate-co-ethylene dimethacrylate) (HEMA-EDMA) was photografted through a quartz photomask with pentafluoropropyl methacrylate (PFPMA) to create superhydrophobic regions (cf. Experimental procedures). Alternatively, by using thiol-yne chemistry-based modification. Using these methods, arrays of superhydrophilic spots with specific geometry and size can be created. It has been shown that it is possible to create arrays of thousands of identical microdroplets in a single step simply by dipping the substrate into an aqueous solution or rolling a droplet across the array (Fig. 1). The rolling droplet method is useful for printing precious reagents because many spots can be filled without using a large amount of solution. Due to the extreme difference between the superhydrophilicity of the spots and the superhydrophobicity of the barriers, water is easily removed from the barriers but fills the superhydrophilic regions. Examples of arrays of water microdroplets with different geometries and sizes are depicted in Fig. 2A (cf. Fig. 3 for droplet formation using a methyl green solution). Formation of droplets with sharp-edged geometries, such as triangles, is also possible. As can be seen in the images, as soon as the nanoporous superhydrophilic areas are wetted, the underlying polymer becomes transparent due to reduced light scattering caused by matched refractive indexes, allowing easier discrimination of spots and facilitating the use of inverted microscopes. Each microdroplet functions as a small lens with the surface curvature determined by the geometry of the hydrophilic spots (Fig. 2b).

The formation of isolated droplets is reproducible and leads to homogeneous microdroplets. The volumes of the formed droplets depend on the size and geometry of the hydrophilic spots. The smallest microdroplets that could be produced had a volume of 5 to 100 nL using a 100 × 100 µm² square micropattern This was mainly due to the limitations of the resolution using this patterning technique and the inability to measure such small volumes before evaporation occurs. The volume of large droplets is more difficult to control because of the increasingly important role of gravity. This effect was observed for 3 mm × 3 mm square patterns and larger.

### Example 2:

### Deposition of cells present in an aqueous solution

The rapid formation of droplet arrays on the porous substrate can be used as a convenient tool for the deposition of cells present in an aqueous solution. Fig. 2C shows an array of microdroplets containing rat mammary carcinoma cells stably expressing enhanced green fluorescent protein (MTly-CMV-eGFP-neo) immediately after droplet formation. This type of an array combines the advantages of microplates where cells present in individual wells are physically isolated from each other, thereby reducing the problem of cross-contamination, with the advantages of miniaturization and parallelization of cell microarrays. However, contrary to the cell microarray method, the diffusion of small molecules in the case of the DropletArray will be confined to the individual microdroplets, which in turn can save a lot of precious materials and avoid cross-contamination. This one-step method for making arrays of thousands of microdroplets incorporating cells opens a way to a variety of screening applications.

As in the case of cell microarrays, the array of hydrophilic spots can be pre-printed with libraries of small molecules or transfection reagents. The hydrophobic barriers allow different volumes of solution to be printed per spot, while still confining the solution within the defined hydrophilic area. Thus, volume and concentration adjustments can be made without changing the footprint of the printed spot. The formation of cell-encapsulated droplets on the hydrophilic spots will then be affected by the pre-printed solutions and can then be screened.

### Example 3:

### Agglomeration of cells to 3D cell structures:

Seeding of cells is performed in a cell culture dish. A DropletArray is preincubated for 45 min in cell medium containing 1% FCS and 1% Penstrep. After drying the DropletArray is coated with 2.2% Gelatin in water for 45 min. Cells are seeded on this pre-incubated DropletArray in concentrations from 10 to 100 ×10^4 cells/mL by applying a 1.5 ml large Standing Droplet onto the surface. Droplets form while tilting the slide and letting the cell suspension drain off after a sedimentation time of 30 to 90 seconds. After formation of the droplets DropletArray is taken with a pair of forceps and is turned upside down and is placed on the table that is standing in a cell culture dish where the bottom is filled with PBS. Cells are cultured from 1 to 14 days in an incubator with 37°C and 5% CO₂. Formation of spheroids occur within 24-96 hours depending on the cell line. For analysis of the spheroids the DropletArray is taken of the table, turned back to its normal position and is placed in a fresh cell culture dish to perform microscopic imaging. Afterwards it is placed pack on the table in the upside down position and placed back in the incubator. Harvesting of the cells is done by dipping the DropletArray containing spheroids into a solution of cell medium or PBS where they sink from their location in the droplet to the solution.

## Claims

1. A method of producing 3D cell structures, comprising the steps of:
(1) forming an array of separated homogenous aqueous fluid microdroplets of a desired shape and size in a desired spatial pattern, wherein the separated homogenous aqueous fluid microdroplets comprise cells, wherein the forming of the array comprises:
(a) providing a patterned substrate with a solid support coated with a layer or film comprising
(i) hydrophilic areas having the desired shape and size; surrounded by
(ii) hydrophobic areas separating the hydrophilic areas into the desired spatial pattern;
and
(b) applying the aqueous fluid comprising the cells to a multitude of the hydrophilic areas at the same time;
(2) turning the patterned substrate with the solid support upside down such that the separated homogeneous aqueous fluid microdroplets form hanging separated homogeneous aqueous fluid microdroplets;
(3) agglomerating the cells comprised in the hanging separated homogeneous aqueous fluid microdroplets to form 3D cell structures; and
further comprising a step (2') between step (2) and (3):
(2') providing the turned patterned substrate with the solid support comprising the hanging separated homogeneous aqueous fluid microdroplets on a holder, wherein the holder comprises a plate having an opening which is adjusted to hold the turned patterned substrate with the solid support;
wherein a cell-culture dish comprising a liquid is provided below the holder,
wherein the volume of one hanging separated homogenous aqueous fluid microdroplet is in the range from 5 nL to 100 nL, and
wherein the holder is a table comprising a tabletop with an opening which is adjusted to hold the turned patterned substrate with the solid support and comprises legs which extend from the surface of the plate facing downwards to the ground.

2. The method according to claim 1, wherein the 3D cell structures are selected from spheroids, organoids, and embryonic bodies, with the proviso that the embryonic bodies are not human embryonic bodies and no human embryo is destroyed in the method.

3. The method according to claim 1 or 2, wherein the cells are selected from at least one member of a group consisting of human, animal or plant primary cells, immortalized adherent and non-adherent cell lines, human and animal cancer cells, human and animal stem cells.

4. The method according to any one of claims 1 to 3, wherein the aqueous fluid is selected from the group consisting of water, aqueous solutions, and aqueous media.

5. The method according to any one of claims 1 to 4, wherein the 3D cell structures are located in the lower end of the hanging separated homogenous aqueous fluid microdroplets.

6. The method according to any one of claims 1 to 5, wherein each of the hanging separated homogenous aqueous fluid microdroplets comprises one 3D cell structure.

7. A hanging droplet device comprising
an array of hanging separated homogenous aqueous fluid microdroplets of a desired shape and size in a desired spatial pattern, wherein the hanging separated homogenous aqueous fluid microdroplets comprise 3D cell structures, hanging from a patterned substrate with a solid support coated with a layer or film, wherein the patterned substrate comprises
(i) hydrophilic areas having the desired shape and size; surrounded by
(ii) hydrophobic areas separating the hydrophilic areas into the desired spatial pattern, and
wherein the patterned substrate faces down,
wherein the patterned substrate with the solid support is provided on a holder comprising a plate having an opening which is adjusted to hold the patterned substrate with the solid support,
wherein a cell-culture dish comprising a liquid is provided below the holder,
wherein the volume of one hanging separated homogenous aqueous fluid microdroplet is in the range from 5 nL to 100 nL, and
wherein the holder is a table comprising a tabletop with an opening which is adjusted to hold the turned patterned substrate with the solid support and comprises legs which extend from the surface of the plate facing downwards to the ground.

8. Use of the hanging droplet device according to claim 7 for high-throughput screening of the 3D cell structures.

9. Use of the hanging droplet device according to claim 7 for cultivating 3D cell structures.

## Patentansprüche

1. Verfahren zur Herstellung von 3D-Zellstrukturen, umfassend die Schritte:
(1) Bildung einer Gruppierung von getrennten homogenen wässrigen Fluid-Mikrotröpfchen mit einer vorgesehenen Form und Größe in einer vorgesehenen räumlichen Struktur, wobei die getrennten homogenen wässrigen Fluid-Mikrotröpfchen Zellen umfassen, wobei das Bilden der Gruppierung umfasst:
(a) Bereitstellen eines strukturierten Substrats mit einem festen Träger, der mit einer Schicht oder einem Film beschichtet ist, die oder der
(i) hydrophile Bereiche umfasst, welche die vorgesehene Form und Größe aufweisen; die durch
(ii) hydrophobe Bereiche umgeben sind, welche die hydrophilen Bereiche in die vorgesehene räumliche Struktur aufteilen;
und
(b) Aufbringen des wässrigen Fluids, das die Zellen umfasst, auf eine Vielzahl der hydrophilen Bereiche gleichzeitig;
(2) Umdrehen des strukturierten Substrats mit dem festen Träger, so dass die getrennten homogenen wässrigen Fluid-Mikrotröpfchen hängende getrennte homogene wässrige Fluid-Mikrotröpfchen bilden;
(3) Agglomerieren der Zellen, die in den hängenden getrennten homogenen wässrigen Fluid-Mikrotröpfchen enthalten sind, zur Bildung von 3D-Zellstrukturen; und
ferner umfassend einen Schritt (2') zwischen dem Schritt (2) und (3):
(2') Bereitstellen des umgedrehten strukturierten Substrats mit dem festen Träger, der die hängenden getrennten homogenen wässrigen Fluid-Mikrotröpfchen umfasst, auf einem Halter, wobei der Halter eine Platte mit einer Öffnung umfasst, die zum Halten des umgedrehten strukturierten Substrats mit dem festen Träger ausgebildet ist;
wobei eine Zellkulturschale, die eine Flüssigkeit umfasst, unterhalb des Halters bereitgestellt ist,
wobei das Volumen eines hängenden getrennten homogenen wässrigen Fluid-Mikrotröpfchens im Bereich von 5 nL bis 100 nL liegt, und
wobei der Halter ein Tisch ist, der eine Tischplatte mit einer Öffnung umfasst, die zum Halten des umgedrehten strukturierten Substrats mit dem festen Träger ausgebildet ist und Füße umfasst, die sich von der Oberfläche der Platte erstrecken und abwärts zum Boden gerichtet sind.

2. Verfahren nach Anspruch 1, wobei die 3D-Zellstrukturen aus Sphäroiden, Organoiden und embryonischen Körpern ausgewählt sind, mit der Maßgabe, dass die embryonischen Körper keine menschlichen embryonischen Körper sind und in dem Verfahren kein menschlicher Embryo zerstört wird.

3. Verfahren nach Anspruch 1 oder 2, wobei die Zellen aus mindestens einem Element einer Gruppe ausgewählt sind, die aus menschlichen, tierischen oder pflanzlichen Primärzellen, immortalisierten adhärenten und nicht-adhärenten Zelllinien, menschlichen und tierischen Krebszellen, menschlichen und tierischen Stammzellen besteht.

4. Verfahren nach einem der Ansprüche 1 bis 3, wobei das wässrige Fluid aus der Gruppe, bestehend aus Wasser, wässrigen Lösungen und wässrigen Medien ausgewählt ist.

5. Verfahren nach einem der Ansprüche 1 bis 4, wobei sich die 3D-Zellstrukturen in dem unteren Ende der hängenden getrennten homogenen wässrigen Fluid-Mikrotröpfchen befinden.

6. Verfahren nach einem der Ansprüche 1 bis 5, wobei jedes der hängenden getrennten homogenen wässrigen Fluid-Mikrotröpfchen eine 3D-Zellstruktur umfasst.

7. Vorrichtung mit hängenden Tröpfchen, die
eine Gruppierung von hängenden getrennten homogenen wässrigen Fluid-Mikrotröpfchen mit einer vorgesehenen Form und Größe in einer vorgesehenen räumlichen Struktur umfasst, wobei die hängenden getrennten homogenen wässrigen Fluid-Mikrotröpfchen 3D-Zellstrukturen umfassen und von einem strukturierten Substrat mit einem festen Träger hängen, der mit einer Schicht oder einem Film beschichtet ist, wobei das strukturierte Substrat
(i) hydrophile Bereiche umfasst, welche die vorgesehene Form und Größe aufweisen; die durch
(ii) hydrophobe Bereiche umgeben sind, welche die hydrophilen Bereiche in die vorgesehene räumliche Struktur aufteilen;
und
wobei das strukturierte Substrat abwärts gerichtet ist,
wobei das strukturierte Substrat mit dem festen Träger auf einem Halter bereitgestellt ist, wobei der Halter eine Platte mit einer Öffnung umfasst, die zum Halten des strukturierten Substrats mit dem festen Träger ausgebildet ist,
wobei eine Zellkulturschale, die eine Flüssigkeit umfasst, unterhalb des Halters bereitgestellt ist,
wobei das Volumen eines hängenden getrennten homogenen wässrigen Fluid-Mikrotröpfchens im Bereich von 5 nL bis 100 nL liegt, und
wobei der Halter ein Tisch ist, der eine Tischplatte mit einer Öffnung umfasst, die zum Halten des umgedrehten strukturierten Substrats mit dem festen Träger ausgebildet ist und Füße umfasst, die sich von der Oberfläche der Platte erstrecken und abwärts zum Boden gerichtet sind.

8. Verwendung der Vorrichtung mit hängenden Tröpfchen nach Anspruch 7 für ein Hochdurchsatz-Screening der 3D-Zellstrukturen.

9. Verwendung der Vorrichtung mit hängenden Tröpfchen nach Anspruch 7 zum Kultivieren von 3D-Zellstrukturen.

## Revendications

1. Procédé de production de structures cellulaires tridimensionnelles, comprenant les étapes consistant à :
(1) former un réseau de micro gouttelettes de fluide aqueux homogènes séparées d'une forme et taille souhaitées en un motif spatial souhaité, dans lequel les microgouttelettes de fluide aqueux homogènes séparées comprennent des cellules, dans lequel la formation du réseau comprend :
(a) pourvoir un substrat à motif d'un support solide enduit d'une couche ou d'un film comprenant
(i) des zones hydrophiles ayant la forme et taille souhaitées ; entourées de
(ii) zones hydrophobes séparant les zones hydrophiles en le motif spatial souhaité ;
et
(b) appliquer le fluide aqueux comprenant les cellules à une multitude des zones hydrophiles en même temps ;
(2) retourner le substrat à motif avec le support solide de sorte que les micro gouttelettes de fluide aqueux homogènes séparées forment des micro gouttelettes de fluide aqueux homogènes séparées en suspension ;
(3) agglomérer les cellules comprises dans les microgouttelettes de fluide aqueux homogènes séparées en suspension pour former des structures cellulaires tridimensionnelles ; et
comprenant en outre une étape (2') entre l'étape (2) et (3) :
(2') prévoir le substrat à motif retourné avec le support solide comprenant les microgouttelettes de fluide aqueux homogènes séparées en suspension sur une monture, dans lequel la monture comprend une plaque ayant une ouverture qui est ajustée pour contenir le substrat à motif retourné avec le support solide ;
dans lequel une boîte de culture cellulaire comprenant un liquide est prévue en dessous de la monture,
dans lequel le volume d'une microgouttelette de fluide aqueux homogène séparée en suspension est dans la plage de 5 ni à 100 nl, et
dans lequel la monture est une table comprenant un plateau avec une ouverture qui est ajustée pour contenir le substrat à motif retourné avec le support solide et comprend des jambes qui s'étendent de la surface de la plaque tournée vers le bas au sol.

2. Procédé selon la revendication 1, dans lequel les structures cellulaires tridimensionnelles sont sélectionnées parmi des sphéroïdes, organoïdes et corps embryonnaires, à condition que les corps embryonnaires ne soient pas des corps embryonnaires humains et qu'aucun embryon humain n'ait été détruit au cours du procédé.

3. Procédé selon la revendication 1 ou 2, dans lequel les cellules sont sélectionnées parmi au moins un membre d'un groupe constitué de cellules primaires humaines, animales ou végétales, de lignées cellulaires adhérentes et non adhérentes immortalisées, de cellules cancéreuses humaines et animales, de cellules souches humaines et animales.

4. Procédé selon l'une quelconque des revendications 1 à 3, dans lequel le fluide aqueux est sélectionné parmi le groupe constitué de l'eau, de solutions aqueuses et de milieux aqueux.

5. Procédé selon l'une quelconque des revendications 1 à 4, dans lequel les structures cellulaires tridimensionnelles sont situées dans l'extrémité inférieure des microgouttelettes de fluide aqueux homogènes séparées en suspension.

6. Procédé selon l'une quelconque des revendications 1 à 5, dans lequel chacune des micro gouttelettes de fluide aqueux homogènes séparées en suspension comprend une structure cellulaire tridimensionnelle.

7. Dispositif de gouttelettes en suspension comprenant
un réseau de microgouttelettes de fluide aqueux homogènes séparées en suspension d'une forme et taille souhaitées en un motif spatial souhaité, dans lequel les microgouttelettes de fluide aqueux homogènes séparées en suspension comprennent des structures cellulaires tridimensionnelles, en suspension depuis un substrat à motif avec un support solide enduit d'une couche ou d'un film, dans lequel le substrat à motif comprend
(i) des zones hydrophiles ayant la forme et taille souhaitées ; entourées de
(ii) zones hydrophobes séparant les zones hydrophiles en le motif spatial souhaité, et
dans lequel le substrat à motif est tourné vers le bas,
dans lequel le substrat à motif avec le support solide est prévu sur une monture comprenant une plaque ayant une ouverture qui est ajustée pour contenir le substrat à motif avec le support solide,
dans lequel une boîte de culture cellulaire comprenant un liquide est prévue en dessous de la monture,
dans lequel le volume d'une microgouttelette de fluide aqueux homogène séparée en suspension est dans la plage de 5 nl à 100 nl, et
dans lequel la monture est une table comprenant un plateau avec une ouverture qui est ajustée pour contenir le substrat à motif retourné avec le support solide et comprend des jambes qui s'étendent de la surface de la plaque tournée vers le bas au sol.

8. Utilisation du dispositif de gouttelettes en suspension selon la revendication 7 pour un criblage à haut débit des structures cellulaires tridimensionnelles.

9. Utilisation du dispositif de gouttelettes en suspension selon la revendication 7 pour la culture de structures cellulaires tridimensionnelles.
